# EUROPEAN PATENT APPLICATION

(11) **EP 3 741 775 A1**
(43) Date of publication of application: **25.11.2020**
(21) Application number: 19738798.8
(22) Date of filing: 10.01.2019
(51) Int. Cl.: C07K 16/00, C07K 16/06, C07K 1/18, C07K 1/34

(54) **PRODUCTION PROCESS FOR IMMUNOGLOBULIN FOR INTRAVENOUS INJECTION**

(30) Priority: 15.01.2018 CN 201810036376
(71) Applicant: SICHUAN YUANDA SHUYANG PHARMACEUTICAL CO., LTD., Chengdu, Sichuan 610000 (CN)
(72) Inventor: ZHENG, Yan, Chengdu, Sichuan 610000 (CN); WANG, Wei, Chengdu, Sichuan 610000 (CN); ZHANG, Feiguan, Chengdu, Sichuan 610000 (CN); BAI, Ting, Chengdu, Sichuan 610000 (CN); ZHENG, Xianjiao, Chengdu, Sichuan 610000 (CN)
(74) Representative: Maiwald Patent- und Rechtsanwaltsgesellschaft mbH
(86) International application number: PCT/CN2019/071165
(87) International publication number: WO 2019/137435

(57) **Abstract**

Provided in the present invention is a production process of intravenous immunoglobulin, which comprises fractionation of healthy human plasma to obtain fraction I+II+III/II+III; then precipitating by means of octanoic acid and filtration to obtain a clear liquid; passing the clear liquid through the following chromatographies sequentially: anion chromatography A, anion chromatography B, and affinity chromatography C; performing ultra-filtration concentration or dilution to the flow-through and washing solution after chromatography and then formulating, next filtering by means of a nanofilter, incubating at a low pH, mixing after incubation, formulating and sterilizing to obtain an intravenous immunoglobulin. The production process provided by the present invention may obtain an IgG product having high yield and purity, and more importantly, the anti-A, anti-B index and ACA index of the obtained products are greatly reduced, which meets the requirements in "Chinese Pharmacopoeia", while the osmotic pressure molar concentration of the sample also meets the requirements.

## Description

### FIELD

The present invention relates to the field of biopharmaceuticals, in particular to a method of preparing an intravenous immunoglobulin.

### BACKGROUND

Immunoglobulin (Ig) is an important part of the human immune system, which is a type of animal protein with antibody activity and plays an important role in the human body to recognize bacterias, viruses and activate complement. The immunoglobulins in human plasma can be divided into five categories including IgG, IgA, IgM, IgD and IgE, of which the IgG is the main one. The most remarkable feature of IgG is the ability of specifically recognizing antigen and combining with it to form a complex. After the complex is phagocytosed by phagocytes, the antigen loses its toxicity and pathogenicity. Since Cohn developed an ethanol precipitation method, human plasma enriched with immunoglobulins has been widely studied and used for the treatment of various infections or congenital defects.

At present, the immunoglobulins obtained at domestic and overseas are mainly purified from plasma or fraction II, and few are extracted from fraction I+II+III or fraction II+III due to the complex composition of the fraction I+II+III or the fraction II+III which makes separation and purification difficult, and difficult to analyze.

The immunoglobulin, especially an IgG concentrate, can be obtained by various methods, such as polyethylene glycol precipitation, protease hydrolysis and the like in addition to the selective protein precipitation by ethanol. Generally, the method for preparing the IgG concentrate is low-temperature ethanol centrifugation (i.e. pressure filtration), with low product yield, low purity and poor stability.

In order to improve the yield and purity of IgG product, researchers at home and aboard have performed a lot of research work, mainly using two-step pressure filtration in combination with two-step chromatography or one-step chromatography, by which high yield and high purity are substantively obtained. A Chinese patent (CN 103554253 B) discloses a method of preparing a human intravenous immunoglobulin, including separation of fraction II+III by two-step pressure filtration, removal of the fraction III by pressure filtration via low-temperature ethanol, and then obtaining the immunoglobulin by two-step chromatography, in which the immunoglobulin obtained has a high biological activity, a high yield (not less than 6.5 g/L plasma) and a high purity (99% or above), however, this patent does not detect anti-A and anti-B antibodies, thus the IgG product obtained has a high content of anti-A and anti-B antibodies which easily goes beyond the limitation ruled in the Chinese Pharmacopoeia without using specific gels to remove the anti-A and anti-B antibodies. Another Chinese patent (CN 103394084 B) provides an immunoglobulin IgG concentrate that contains polyreactive IgG and removes anti-A and anti-B antibodies, exhibiting favorable anti-A/anti-B antibody removal effect, in which the anti-A antibody in the IgG concentrate is not more than 23 ng/mg, especially between 19 and 23 ng/mg and the anti-B antibody in the IgG concentrate is not more than 20 ng/mg especially between 12 and 20 ng/mg, however, the product yield and purity are not investigated in this patent. Existing research results show that, in order to obtain IgG products with a higher yield and purity and excellent anti-A/anti-B antibody removal effect, the preparation method has to meet high requirements. The existing methods cannot achieve good results on both the yield and purity and the anti-A/anti-B antibody removal.

In addition, the existing preparation methods further have the following defects that cannot be resolved. For example, the final product obtained after two-step chromatography and nano-membrane filtration of the flow-through liquid displays a high ACA content even though the anti-A and anti-B indicators have already reached the requirement of the Chinese Pharmacopoeia (2010), thus the ACA content cannot meet the standard after incubation. Further, the osmolarity easily causes natural crystallization during the ultrafiltration and formulation after octanoic acid precipitation and two-step chromatography, resulting in unable to detect osmolarity of the product or non-qualified osmolarity, further would lower the yield, generate a long tail for washing peaks and difficulty in cleaning chromatography column. Therefore, it is necessary to provide an IgG preparation method that can solve the above problems, obtaining IgG products which have not only high yield and purity but also anti-A, anti-B and ACA indicators that meet the requirements of the Chinese Pharmacopoeia (2010).

### SUMMARY

The purpose of the present disclosure is to solve the technical problems as described above. The present disclosure in embodiments provides a method of preparing an intravenous immunoglobulin. According to the present method, intravenous immunoglobulin obtained has a high yield and purity, and more importantly, greatly decreased anti-A and anti-B indicators and ACA indicator, excellently meeting the requirements of the Chinese Pharmacopoeia (2010).

For this, the present disclosure in embodiments provides a method of preparing an intravenous immunoglobulin, comprising steps of:
(1) separating fraction I+II+III or fraction II+III from plasma,
(2) octanoic acid precipitation: precipitating the fraction I+II+III or fraction II+III with octanoic acid, followed by filtering to obtain a clarified liquid, in which preferably the octanoic acid is of a concentration of 10 to 30 mM, more preferably 22 to 25 mM,
(3) performing chromatography according to a combination of chromatography A, chromatography B and chromatography C, a combination of chromatography A and chromatography C, or a combination of chromatography B and chromatography C, wherein
   chromatography A: filtration concentrating the clarified liquid to obtain a first protein solution in a protein content of 2 to 20 g/L, preferably 5 to 15 g/L, more preferably 8 to 10 g/L, passing the first protein solution through an anion chromatography column A under a first chromatography condition of pH 4.50 to 5.50, 5 to 20 mM of sodium acetate buffer, 0.5 to 2.0 ms/cm of conductivity and 60 to 150 cm/hour of linear rate for equilibration and loading, preferably pH 4.70 to 5.30, 8 to 16 mM of sodium acetate buffer, 0.8 to 1.8 ms/cm of conductivity and 70 to 120 cm/hour of linear rate for equilibration and loading, more preferably pH 4.8 to 5.0, 12 to 14 mM of sodium acetate buffer, 1.0 to 1.2 ms/cm of conductivity and 90 to 100 cm/hour of linear rate for equilibration and loading,
   the anion chromatography column A comprises Fractogel EMD DEAE anion chromatography column, Capto Q anion chromatography column or UNO sphere Q anion chromatography column, preferably Fractogel EMD DEAE anion chromatography column,
   chromatography B: passing a first flow-through solution and washing solution mixture through an anion chromatography column B under a second chromatography condition of pH 5.50 to 6.50, 5 to 20 mM of sodium acetate buffer, 0.5 to 2.5 ms/cm of conductivity and 60 to 150 cm/hour of linear rate for equilibration and loading, preferably pH 5.70 to 6.30, 8 to 16 mM of sodium acetate buffer, 0.8 to 1.8 ms/cm of conductivity and 70 to 100 cm/hour of linear rate for equilibration and loading, more preferably pH 6.0 to 6.2, 12 to 14 mM of sodium acetate buffer, 1.0 to 1.2 ms/cm of conductivity and 90 to 100 cm/hour of linear rate for equilibration and loading,
   the anion chromatography column B comprises Fractogel EMD TMAE anion chromatography column, CaptoQ XP anion chromatography column or Nuvia HR Q anion chromatography column, preferably Fractogel EMD TMAE anion chromatography column,
   chromatography C: ultrafiltration concentration of a second flow-through solution and washing solution mixture with a buffer to obtain a second protein solution in a protein content of 20 to 60 g/L, preferably 30 to 50g/L, more preferably 35 to 45g/L, in which the buffer comprises acetate or citrate or phosphate at pH 4.50 to 7.30and NaCl, preferably 5 to 20 mM of acetate or citrate or phosphate at pH 5.00 to 6.00 and 80 to 150 mM of NaCl, more preferably acetate or citrate or phosphate at pH 5.30 to 5.50 and 100 to 120 mM of NaCl, ultrafiltration dialysis of the second protein solution to allow the pH to be consistent with the buffer, thus obtaining a third protein solution, passing the third protein solution through an affinity chromatography column C under an affinity chromatography condition comprising 60 to 150 cm/hour of linear rate under the buffer condition for equilibration and loading, preferably 70 to 130 cm/hour of linear rate for equilibration and loading, more preferably 90 to 110 cm/hour of linear rate for equilibration and loading,
   the affinity chromatography column C comprises Eshmuno P Anti-A/Anti-B mix affinity chromatography column, Eshmuno P Anti-A affinity chromatography column or Eshmuno P Anti-B affinity chromatography column, and
(4) ultrafiltration concentration or dilution, viral inactivation, formulation and filling to obtain the intravenous immunoglobulin, in which preferably the formulation is conducted by using glycine or proline.

According to the preparation method of the present disclosure, octanoic acid precipitation and chromatography conditions are key factors, specifically the octanoic acid concentration, protein content, and indicators including pH value, conductivity and buffer in the chromatography all display an important impact on chromatography results, and the immunoglobulin IgG after purification shows a big difference in some indicators due to the different chromatography conditions. Through a lot of comparative experiments, the present disclosure shows that the chromatography conditions as described above can ensure that the immunoglobulin IgG has excellent anti-A, anti-B and ACA indicators. Especially, under the preferred scheme of the present invention, Anti-A = 1: 4, Anti-B = 1: 4, ACA = 2%, and the osmolarity can reach 285 mOsmol/kg, PKA <10 IU / mL, all meeting the requirements of the Chinese Pharmacopoeia (2010). When the chromatography conditions are beyond the scope of the present disclosure, the anti-A and anti-B indicators cannot be removed to reach the excellent level of the present disclosure, further the product yield also cannot reach the level of the present disclosure. In particular, the buffer conditions used in the present disclosure exhibit a significant effect on the indicators of product, such as yield and osmolarity. The osmolarity of the product can excellently meet the standard within the buffer conditions of the present disclosure, while the osmolarity of the product cannot reach the standard and easily causes natural crystallization outside the buffer conditions of the present disclosure.

In the chromatography A of step (3) of the present disclosure, the chromatography process is performed by using an anion chromatography column A, mainly including Fractogel EMD DEAE, Capto Q or UNO sphere Q anion chromatography column, and all of three can achieve good anti-A and anti-B indicators and ACA indicator. Among them, the Fractogel EMD DEAE anion chromatography column is most preferred, which can achieve a higher yield of immunoglobulin when used under the chromatography conditions of the present disclosure, while the Capto Q and UNO sphere Q anion chromatography columns achieve a lower yield of immunoglobulin compared to the Fractogel EMD DEAE anion chromatography column.

In the chromatography B of step (3) of the present disclosure, the chromatography process is performed by using an anion chromatography column B, mainly including Fractogel EMD TMAE, CaptoQ XP or Nuvia HR Q anion chromatography column, and all of three can achieve good anti-A and anti-B indicators and ACA indicator. Among them, the Fractogel EMD TMAE anion chromatography column is most preferred, which can achieve a higher yield of immunoglobulin when used under the chromatography conditions of the present disclosure, while the CaptoQ XP and Nuvia HR Q anion chromatography columns achieve a lower yield of immunoglobulin compared to the Fractogel EMD TMAE anion chromatography column.

In step (3) of the present disclosure, the chromatography can be performed according to the following means: 1) three-step chromatography in the sequence of chromatography A, chromatography B and chromatography C, 2) two-step chromatography in the sequence of chromatography A and chromatography C, or 3) two-step chromatography in the sequence of chromatography B and chromatography C. The chromatography methods as described above can all remove anti-A and anti-B significantly.

As an embodiment of the present disclosure, the anion chromatography column A in the chromatography A is XK50/30 chromatography column filled with corresponding anion chromatography gels to a height of 18 to 23 cm and has a gel load of 300 to 700 g precipitates per 400 mL gels, preferably 350 to 650 g precipitates per 400 mL gels, more preferably 450 to 550 g precipitates per 400 mL gels.

As an embodiment of the present disclosure, the anion chromatography column B in the chromatography B is XK50/30 chromatography column filled with corresponding anion chromatography gels to a height of 18 to 23 cm and has a gel load of 300 to 700 g precipitates per 400 mL gels, preferably 350 to 650 g precipitates per 400 mL gels, more preferably 450 to 550 g precipitates per 400 mL gels.

Gel load can have a certain influence on the effect of chromatography. The present disclosure finally determined that an excellent chromatography effect can be achieved under the gel load as described above through a lot of experiments and exploration of process conditions.

As an embodiment of the present disclosure, the Eshmuno P Anti-A/Anti-B mix affinity chromatography column, Eshmuno P Anti-A affinity chromatography column or Eshmuno P Anti-B affinity chromatography column in the chromatography C is XK 16/20 chromatography column filled with Eshmuno P Anti-A affinity gels and/or Eshmuno P Anti-B affinity gels to a height of 5 to 25 cm, preferably a height of 6 to 20 cm, the Eshmuno P Anti-A/Anti-B mix affinity chromatography column is filled with Eshmuno P Anti-A affinity gels and Eshmuno P Anti-B affinity gels in a ratio of 1 to 2: 2 to 3.

In chromatography C, a product with a high yield of immunoglobulin can be obtained no matter whether a mixed affinity chromatography column or a single affinity chromatography column is used, further the anti-A or anti-B indicator in the product can fully meet the standard of the Chinese Pharmacopoeia (2010).

The method of the present disclosure further comprises that in the chromatography B, the first flow-through solution and washing solution mixture is subjected to pH adjustment with a buffer, stilled at a temperature of 2 to 8°C for 1 hour or above and filtered to get a supernatant before passing through the anion chromatography column B.

Further, prior to separating fraction I+II+III or fraction II+III from plasma in step (1), the method further comprises treating the plasma from a healthy subject with ethanol at a low temperature to obtain a precipitate of the fraction I+II+III or fraction II+III.

Further, after being obtained, the fraction I+II+III or fraction II+III is dissolved with 10 to 25 times of water for injection (WFI) in steps at a temperature of 2 to 25 °C and adjusted to pH 4.00 to 5.00, with stirring for 1 hour or above.

Further, after dissolving with WFI in steps, the method further comprises adjusting the dissolved solution with NaOH to pH 4.80 to 5.80, adding the octanoic acid dropwise to a final concentration of 10 to 30 mM, and then adjusting with NaOH back to pH 4.80 to 5.80, followed by stirring for 1 hour or above and filtering to obtain the clarified liquid after octanoic acid precipitation.

Further, ultrafiltration concentration or dilution in step (4) further comprises ultrafiltration concentrating or diluting a third flow-through solution and washing solution mixture to obtain a fourth protein solution in a protein content of 5 to 100 g/L, adding 1 to 5% (w/w) glycine or proline, and adjusting pH to 3.8 to 4.4 before the formulation.

Further, the viral inactivation comprises: filtering through a nano-membrane of 20 nm or 15 nm, combining and sterilizing the filtered solution, and then incubating at 25 °C under low pH, followed by mixing and formulating the resulting solution, and filling after sterilization to obtain the intravenous immunoglobulin, or
incubating at 25 °C under low pH, filtering through a nano-membrane of 20 nm or 15 nm, followed by mixing and formulating the filtered solution, and filling after sterilization to obtain the intravenous immunoglobulin.

The present disclosure has the following beneficial effects.

After the octanoic acid treatment, three-step chromatography treatment (chromatography A, chromatography B and chromatography C) or two-step chromatography treatment (chromatography A and chromatography C) or two-step chromatography treatment (chromatography B and chromatography C), and then ultrafiltration and formulation according to the present disclosure, the total IgG yield can reach 90.9% or above, the purity is 99.9 % or above, the total protein is about 56.69 g, the IgA, IgM and albumin in the finished product are completely removed, the appearance and thermal stability are qualified, the osmolarity is 285 mOsmol/kg, meeting the standards of the Chinese Pharmacopoeia (2010). After the chromatography C treatment, Anti-A=1:4, Anti-B=1:4, ACA=2%, PKA < 10 IU/mL, all of which meet the requirements of the Chinese Pharmacopoeia (2010).

### DESCRIPTION OF DRAWINGS

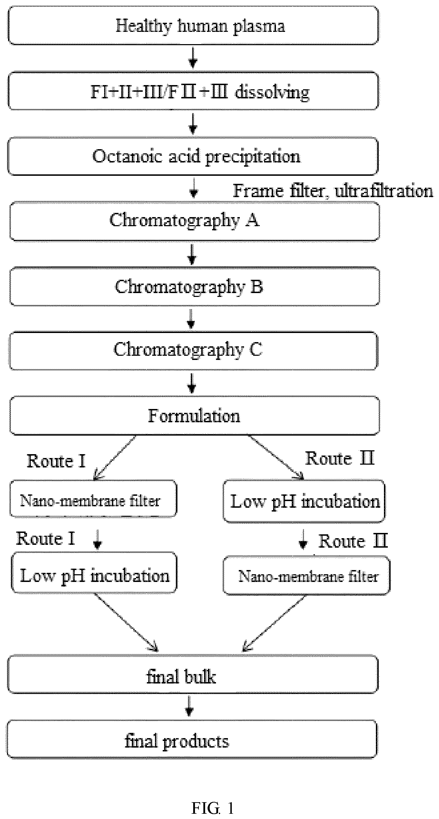

FIG. 1 is a flow chart of the production process of an intravenous immunoglobulin of the present invention.

### DETAILED DESCRIPTION

The present invention is described in detail with reference to the embodiments as below to make the purpose, technical solutions and advantages of the present invention clearer. It is necessary to indicate that the following embodiments are only used to explain and describe the present invention, which is not to limit the present invention. Some non-essential improvements and adjustments made by those skilled in the art based on the summary as described above still fall within the protection scope of the present invention.

As known to skilled in the art, "WFI" is an abbreviation for "water for injection".

In the following examples, the quality testing indicators of immunoglobulin products for intravenous injection are shown in Table 1.

**Table 1**

| **Indicators** | **Standard of Chinese Pharmacopoeia** | **Standard of European Pharmacopoeia** |
|---|---|---|
| protein content | ≥ 50 g/L | ≥30 g/L, 90 to 110 % |
| purity | ≥ 95.0 % | ≥95.0 % |
| molecule distribution | IgG monomer and dimer ≥95.0 % | IgG monomer and dimer ≥90.0 % |
| PKA | ≤35 IU/mL | ≤35 IU/mL |
| ACA | ≤50 % | ≤50 % |
| Anti-A, Anti-B hemagglutinin | ≤1:64 | ≤1:64 |

### Example 1

A method of preparing an intravenous immunoglobulin was performed according to the production process flow of route I in FIG. 1, specifically including the following steps of:
(1) treating a plasma sample from a healthy subject with ethanol at a low temperature to obtain fraction I+II+III, dissolving the fraction I+II+III with 10 times of water for injection (WFI) in steps at a temperature of 2 °C and adjusted to pH 4.00 with acetic acid, with stirring for 1 hour;
(2) octanoic acid precipitation: adjusting the dissolved solution with NaOH to pH 4.80, adding the octanoic acid dropwise to a final concentration of 10 mM within 40 minutes, and then adjusting with NaOH back to pH 4.80, followed by stirring for 1 hour and filtering by frame to obtain a clarified liquid after octanoic acid precipitation;
(3) chromatography A: preparing a Fractogel EMD DEAE anion chromatography column by filling Fractogel EMD DEAE anion gels into a XK50/30 chromatography column to a height of 18 cm, with a gel load of 300 g precipitates per 400 mL gels, filtration concentrating the clarified liquid obtained in (2) to obtain a first protein solution in a protein content of 2 g/L, passing the first protein solution through the Fractogel EMD DEAE anion chromatography column under a chromatography condition of pH 4.50, 5 mM of sodium acetate buffer, 0.5 ms/cm of conductivity and 60 cm/hour of linear rate for equilibration and loading,
(4) chromatography B: preparing a Fractogel EMD TMAE anion chromatography column by filling Fractogel EMD TMAE anion gels into a XK50/30 chromatography column to a height of 18 cm, with a gel load of 300 g precipitates per 400 mL gels, passing a first flow-through solution and washing solution mixture obtained in step (3) through the Fractogel EMD TMAE anion chromatography column under a second chromatography condition of pH 5.50, 5 mM of sodium acetate buffer, 0.5 ms/cm of conductivity and 60 cm/hour of linear rate for equilibration and loading,
(5) chromatography C: preparing an Eshmuno P Anti-A/Anti-B mix affinity chromatography column by filling Eshmuno P Anti-A affinity gels and Eshmuno P Anti-B affinity gels in a ratio of 1:2 to a height of 6 cm, ultrafiltration concentrating a second flow-through solution and washing solution mixture obtained in step (4) with a buffer comprising sodium acetate at pH 4.50 and 60 mM NaCl to obtain a second protein solution in protein content of 20 g/L, ultrafiltration dialysis of the second protein solution to allow the pH to be 4.50 thus obtaining a third protein solution, passing the third protein solution through the Eshmuno P Anti-A/Anti-B mix affinity chromatography column for the affinity chromatography at 60 cm/hour of linear rate for equilibration and loading,
(6) formulation: ultrafiltration dialyzing a third flow-through solution and washing solution mixture obtained in step (5) to obtain a fourth protein solution in a protein content of 5 g/L, adding 1% (w/w) glycine, and adjusting to pH 3.8 with hydrochloric acid, followed by filtering through a nano-membrane of 20 nm at a filtration rate of 80.63 g/24 hours, combining and sterilizing the filtered solution, and then incubating at 25 °C under low pH (pH 3.8) for 21 days, followed by mixing and formulating the incubated solution, and filling after sterilization to obtain a concentrate of immunoglobulin IgG for intravenous injection.

A total of about 56.67 g total protein was obtained and the product yield was 7.06 g/L plasma. After the Fractogel EMD DEAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG was 95% or above. After DEAE, TMAE, Anti-A/Anti-B mix chromatography, ultrafiltration and formulation, the total yield of IgG was 88.3%, the purity was 99.8%, Alb, IgA and IgM were all removed fully, the osmolarity was 281 mOsmol/kg, meeting the standards. The molecule distribution of IgG monomer and dimer was 99.2%, Anti-A=1:16, Anti-B=1:8, ACA=10%, PKA<10 IU/mL, all meeting the requirements of the Chinese Pharmacopoeia (2010).

### Example 2

An immunoglobulin product for intravenous injection was prepared according to the production process flow of route II in FIG. 1, and the steps were as follows:
(1) treating healthy human plasma with ethanol at a low temperature to obtain fraction II+III, dissolving the fraction II+III with 25 times of water for injection (WFI) in steps at a temperature of 25 °C and adjusted to pH 5.00 with hydrochloric acid, with stirring for 3 hours;
(2) octanoic acid precipitation: adjusting the dissolved solution with NaOH to pH 5.80, adding the octanoic acid dropwise to a final concentration of 30 mM within 60 minutes, and then adjusting with NaOH back to pH 5.80, followed by stirring for 3 hours and filtering by frame to obtain a clarified liquid after octanoic acid precipitation;
(3) chromatography A: preparing a Fractogel EMD DEAE anion chromatography column by filling Fractogel EMD DEAE anion gels into an XK50/30 chromatography column to a height of 23 cm, with a gel load of 700 g precipitates per 400 mL gels, filtration concentrating the clarified liquid obtained in (2) to obtain a first protein solution in protein content of 20 g/L, passing the first protein solution through the Fractogel EMD DEAE anion chromatography column under a first chromatography condition of pH 5.50, 20 mM of sodium acetate buffer, 2.0 ms/cm of conductivity and 150 cm/hour of linear rate for equilibration and loading,
(4) chromatography B: preparing a Fractogel EMD TMAE anion chromatography column by filling Fractogel EMD TMAE anion gels into a XK50/30 chromatography column to a height of 23 cm, with a gel load of 700 g precipitates per 400 mL gels, passing a first flow-through solution and washing solution mixture obtained in step (3) through the Fractogel EMD TMAE anion chromatography column under a second chromatography condition of pH 6.50, 20 mM of sodium acetate buffer, 2.5 ms/cm of conductivity and 150 cm/hour of linear rate for equilibration and loading,
(5) chromatography C: preparing an Eshmuno P Anti-A/Anti-B mix affinity chromatography column by filling Eshmuno P Anti-A affinity gels and Eshmuno P Anti-B affinity gels in a ratio of 1: 3 to a height of 25 cm, ultrafiltration concentrating a second flow-through solution and washing solution mixture obtained in step (4) with a buffer comprising sodium phosphate at pH 7.30 and 180 mM NaCl to obtain a second protein solution in protein content of 60 g/L, ultrafiltration dialysis of the second protein solution to allow the pH to be 7.30 thus obtaining a third protein solution, passing the third protein solution through the Eshmuno P Anti-A/Anti-B mix affinity chromatography column for affinity chromatography at 150 cm/hour of linear rate for equilibration and loading,
(6) formulation: ultrafiltration concentrating a third flow-through solution and washing solution mixture obtained in step (5) to obtain a fourth protein solution in a protein content of 100 g/L, adding 5% (w/w) proline, and adjusting to pH 4.4 with phosphoric acid, followed by incubating at 25 °C under low pH (pH 4.4) for 19 days, filtering through a nano-membrane of 15 nm at a filtration rate of 140 g/24 hours, mixing and formulating the nano-filtered solution, and filling after sterilization to obtain a concentrate of immunoglobulin IgG for intravenous injection.

A total of about 55.56 g total protein was obtained and the product yield was 6.89 g/L plasma. After the Fractogel EMD DEAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and the single-step yield of IgG was 95% or above. After DEAE, TMAE, Anti-A/Anti-B mix chromatography, ultrafiltration and formulation, the IgG yield was 87.8%, the purity was 99.7%, Alb, IgA and IgM were all removed fully, the osmolarity was 277 mOsmol/kg, meeting the standards. The molecule distribution of IgG monomer and dimer was 99.1%, Anti-A=1:8, Anti-B=1:16, ACA=12%, PKA<10 IU/mL, all meeting the requirements of the Chinese Pharmacopoeia (2010).

### Example 3

An IgG concentrate was prepared by using the fraction I+II+III as a raw material according to the production process flow of route I in FIG. 1 and the specific steps in Example 1, and the difference was described as below.

In step (1), the fraction I+II+III was dissolved with 18 times of water for injection (WFI) in steps at a temperature of 15 °C and adjusted pH to 4.35 with acetic acid, with stirring for 2 hours.

In step (2), the dissolved solution was adjusted with NaOH to pH 5.20, the octanoic acid was added dropwise to a final concentration of 22 mM, and the mixture was stirred for 2 hours before filtration.

In step (3), the load of Fractogel EMD DEAE anion gels was 350 g precipitates per 400 mL gels; the column was filled with the anion gels to a height of 20 cm; the clarified liquid was concentrated through filtration to a protein content of 5 g/L; and the condition of Fractogel EMD DEAE chromatography was pH 4.70, 8 mM of sodium acetate buffer, 0.8 ms/cm of conductivity and 70 cm/hour of linear rate for equilibration and loading.

In step (4), the load of Fractogel EMD TMAE anion gels was 350 g precipitates per 400 mL gels; the column was filled with the anion gels to a height of 20 cm; and the condition of Fractogel EMD TMAE chromatography was pH 5.70, 8 mM of sodium acetate buffer, 0.8 ms/cm of conductivity and 70 cm/hour of linear rate for equilibration and loading.

In step (5), Eshmuno P Anti-A affinity gels and Eshmuno P Anti-B affinity gels were filled in a ratio of 1: 1 to a height of 6 cm; the second flow-through solution and washing solution mixture was ultrafiltration concentrated with a buffer comprising sodium citrate at pH 5. 0 and 80 mM NaCl to a protein content of 30 g/L followed by ultrafiltration dialysis to allow the pH to be 5.0; and the linear rate for equilibration and loading was 70 cm/hour.

In step (6), the third flow-through solution and washing solution mixture was ultrafiltration concentrated to a protein content of 80 g/L, 3% (w/w) glycine was added, and the pH was adjusted with hydrochloric acid to 4.1.

A total of about 57.21 g total protein was obtained and the product yield was 7.12 g/L plasma. After the Fractogel EMD DEAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and the single-step yield of IgG was 95% or above. After DEAE, TMAE, Anti-A/Anti-B mix chromatography, ultrafiltration and formulation, the total yield of IgG was 89.4%, the purity was 99.8%, Alb, IgA and IgM were all removed fully, the osmolarity was 285 mOsmol/kg, meeting the standards. The molecule distribution of IgG monomer and dimer was 98.9%, Anti-A=1:8, Anti-B=1:8, ACA=9%, PKA<10 IU/mL, all meeting the requirements of the Chinese Pharmacopoeia (2010).

### Example 4

An IgG concentrate was prepared by using the fraction II+III as a raw material according to the production process flow of route II in FIG. 1 and the specific steps in Example 2, and the difference was described as below.

In step (1), the fraction II+III was dissolved with 12 times of water for injection (WFI) in steps at a temperature of 6 °C and adjusted to pH 4.80 with acetic acid, with stirring for 2.5 hours.

In step (2), the dissolved solution was adjusted with NaOH to pH 5.60, and the octanoic acid was added dropwise to a final concentration of 25 mM.

In step (3), the load of Fractogel EMD DEAE anion gels was 650 g precipitates per 400 mL gels; the column was filled with the anion gels to a height of 22 cm; the clarified liquid was concentrated through filtration to a protein content of 15 g/L; and the condition of Fractogel EMD DEAE chromatography was pH 5.30, 16 mM of sodium acetate buffer, 1.8 ms/cm of conductivity and 120 cm/hour of linear rate for equilibration and loading.

In step (4), the load of Fractogel EMD TMAE anion gels was 650 g precipitates per 400 mL gels; the column was filled with the anion gels to a height of 22 cm; and the condition of Fractogel EMD TMAE chromatography was pH 6.30, 16 mM of sodium acetate buffer, 1.8 ms/cm of conductivity and 100 cm/hour of linear rate for equilibration and loading.

In step (5), Eshmuno P Anti-A affinity gels and Eshmuno P Anti-B affinity gels were filled in a ratio of 2: 3 to a height of 20 cm; the second flow-through solution and washing solution mixture was ultrafiltration concentrated with a buffer comprising 20 mM sodium citrate at pH 6. 0 and 150 mM NaCl to a protein content of 50 g/L followed by ultrafiltration dialysis to allow the pH to be 6.00; and the linear rate for equilibration and loading was 130 cm/hour.

In step (6), the third flow-through solution and washing solution mixture was diluted to a protein content of 20 g/L, 4% (w/w) proline was added, and the pH was adjusted with hydrochloric acid to 3.9.

A total of about 57.27 g total protein was obtained and the product yield was 7.12 g/L plasma. After the Fractogel EMD DEAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG was 96% or above. After DEAE, TMAE, Anti-A/Anti-B mix chromatography, ultrafiltration and formulation, the total yield of IgG is 89.32%, the purity was 99.8%, Alb, IgA and IgM were all removed fully, the osmolarity is 287 mOsmol/kg, meeting the standards. The molecule distribution of IgG monomer and dimer was 99.5%, Anti-A=1:4, Anti-B=1:8, ACA=7%, PKA<10 IU/mL, all meeting the requirements of the Chinese Pharmacopoeia (2010).

### Example 5

A finished product of immunoglobulin IgG concentrate for intravenous injection was prepared by using the fraction II+III from human plasma as a raw material according to the method in Example 1, and the difference was described as below.

After completion of the Fractogel EMD DEAE anion chromatography in step (3), the Eshmuno P Anti-A/Anti-B affinity chromatography in step (5) was directly performed.

A total of about 55.46 g total protein was obtained and the product yield was 6.94 g/L plasma. After the Fractogel EMD DEAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG was 93% or above. After DEAE, TMAE, Anti-A/Anti-B mix chromatography, ultrafiltration and formulation, the total yield of IgG was 86.3%, the purity was 99.1%, Alb, IgA and IgM were all removed fully, the osmolarity was 268 mOsmol/kg, meeting the standards. The molecule distribution of IgG monomer and dimer was 98.5%, Anti-A=1:16, Anti-B=1:8, ACA=12%, PKA<10 IU/mL, all meeting the requirements of the Chinese Pharmacopoeia (2010).

### Example 6

A finished product of immunoglobulin IgG concentrate for intravenous injection was prepared by using fraction I+II+III from human plasma as a raw material according to the method in Example 2, and the difference was described as below.

After completion of the octanoic acid precipitation in step (2), the chromatography operations in step (4) and step (5) were directly performed.

A total of about 54.35 g total protein was obtained and the product yield was 6.85 g/L plasma. After the Fractogel EMD TMAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG is 94% or above. After DEAE, TMAE, Anti-A/Anti-B mix chromatography, ultrafiltration and formulation, the total yield of IgG was 89.1%, the purity was 99.4%, Alb, IgA and IgM were all removed fully, the osmolarity was 263 mOsmol/kg, meeting the standards. The molecule distribution of IgG monomer and dimer was 99.2%, Anti-A=1:16, Anti-B=1:8, ACA=14%, PKA<10 IU/mL, all meeting the requirements of the Chinese Pharmacopoeia (2010).

### Example 7

A finished product of immunoglobulin IgG concentrate for intravenous injection was prepared by using fraction II+III from human plasma as a raw material according to the method in Example 3, and the difference was described as below.

In step (1), fraction II+III was obtained and then dissolved.

In step (6), 3% (w/w) glycine was added, and the pH was adjusted with hydrochloric acid to 4.1. After that, the protein solution was incubated at 25 °C under low pH (pH 4.1) for 21 days, filtered through a nano-membrane of 20 nm, followed by mixing and formulating the filtered solution, and filling after sterilization to obtain the immunoglobulin IgG concentrate for intravenous injection.

A total of about 57.73 g total protein was obtained and the product yield was 7.11 g/L plasma. After the Fractogel EMD DEAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG was 97% or above. After DEAE, TMAE, Anti-A/Anti-B mix chromatography, ultrafiltration and formulation, the total yield of IgG was 91.3%, the purity was 99.9%, Alb, IgA and IgM were all removed fully, the osmolarity was 287 mOsmol/kg, meeting the standards. The molecule distribution of IgG monomer and dimer was 99.8%, Anti-A=1:4, Anti-B=1:4, ACA=3%, PKA<10 IU/mL, all meeting the requirements of the Chinese Pharmacopoeia (2010).

### Example 8

An IgG concentrate was prepared by using fraction I+II+III from human plasma as a raw material according to the method in Example 4, and the difference was described as below.

In step (1), fraction I+II+III was obtained and then dissolved.

In step (3), the load of Fractogel EMD DEAE anion gels was 450 g precipitates per 400 mL gels; the column was filled with the anion gels to a height of 20 cm; the clarified liquid was concentrated through filtration to a protein content of 8 g/L; and the condition of Fractogel EMD DEAE chromatography was pH 4.80, 12 mM of sodium acetate buffer, 1.0 ms/cm of conductivity and 90 cm/hour of linear rate for equilibration and loading.

In step (4), the load of Fractogel EMD TMAE anion gels was 450 g precipitates per 400 mL gels; the column was filled with the anion gels to a height of 20 cm; and the condition of Fractogel EMD TMAE chromatography was pH 6.00, 12 mM of sodium acetate buffer, 1.0 ms/cm of conductivity and 90 cm/hour of linear rate for equilibration and loading.

In step (5), the second flow-through solution and washing solution mixture was ultrafiltration concentrated with a buffer comprising 10 mM sodium citrate at pH 5.30 and 100 mM NaCl to a protein content of 35 g/L followed by ultrafiltration dialysis to allow the pH to be 5.30; and the linear rate for equilibration and loading was 90 cm/hour.

A total of about 56.69 g total protein was obtained and the product yield was 7.09 g/L plasma. After the Fractogel EMD DEAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG was 97% or above. After DEAE, TMAE, Anti-A/Anti-B mix chromatography, ultrafiltration and formulation, the total yield of IgG was 90.9%, the purity was 99.9%, Alb, IgA and IgM were all removed fully, and the osmolarity was 285 mOsmol/kg, meeting the standards. The molecule distribution of IgG monomer and dimer was 99.9%, Anti-A=1:4, Anti-B=1:4, ACA=2%, PKA<10 IU/mL, all meeting the requirements of the Chinese Pharmacopoeia (2010).

### Example 9

A finished product of immunoglobulin IgG concentrate for intravenous injection was prepared by using fraction II+III from human plasma as a raw material according to the method in Example 4, and the difference was described as below.

In step (3), the load of Fractogel EMD DEAE anion gels was 550 g precipitates per 400 mL gels; the column was filled with the anion gels to a height of 20 cm; the clarified liquid was concentrated through filtration to a protein content of 10 g/L; and the condition of Fractogel EMD DEAE chromatography was pH 5.00, 14 mM of sodium acetate buffer, 1.2 ms/cm of conductivity and 100 cm/hour of linear rate for equilibration and loading.

In step (4), the load of Fractogel EMD TMAE anion gels was 450 g precipitates per 400 mL gels; the column was filled with the anion gels to a height of 20 cm; and the condition of Fractogel EMD TMAE chromatography was pH 6.00, 12 mM of sodium acetate buffer, 1.0 ms/cm of conductivity and 90 cm/hour of linear rate for equilibration and loading.

In step (5), the second flow-through solution and washing solution mixture was ultrafiltration concentrated with a buffer comprising 15 mM sodium citrate at pH 5.50 and 120 mM NaCl to a protein content of 45 g/L followed by ultrafiltration dialysis to allow the pH to be 5.50; and the linear rate for equilibration and loading is 110 cm/hour.

A total of about 55.51 g total protein was obtained and the product yield was 6.96 g/L plasma. After the Fractogel EMD DEAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG was 95% or above. After DEAE, TMAE, Anti-A/Anti-B mix chromatography, ultrafiltration and formulation, the total yield of IgG was 90.1%, the purity is 99.8%, Alb, IgA and IgM were all removed fully, and the osmolarity was 271 mOsmol/kg, meeting the standards. The molecule distribution of IgG monomer and dimer was 99.8%, Anti-A=1:4, Anti-B=1:4, ACA=5%, PKA<10 IU/mL, all meeting the requirements of the Chinese Pharmacopoeia (2010).

### Example 10

A finished product of immunoglobulin IgG concentrate for intravenous injection was prepared by using fraction II+III from human plasma as a raw material according to the method in Example 5, and the difference was described as below.

In step (6), after addition of glycine and pH adjusting, the protein solution was incubated at 25 °C under low pH (pH 4.4) for 20 days, filtered through a nano-membrane of 15 nm, followed by mixing and formulating the filtered solution, and filling after sterilization to obtain the immunoglobulin IgG concentrate for intravenous injection.

A total of about 55.13 g total protein was obtained and the product yield was 6.91 g/L plasma. After the Fractogel EMD DEAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG was 93% or above. After DEAE, TMAE, Anti-A/Anti-B mix chromatography, ultrafiltration and formulation, the total yield of IgG was 87.5%, the purity was 99.2%, Alb, IgA and IgM were all removed fully, and the osmolarity was 267 mOsmol/kg, meeting the standards. The molecule distribution of IgG monomer and dimer was 98.3%, Anti-A=1:16, Anti-B=1:8, ACA=12%, PKA<10 IU/mL, all meeting the requirements of the Chinese Pharmacopoeia (2010).

A total of about 53.85 g total protein was obtained and the product yield was 6.81 g/L plasma. After the Fractogel EMD TMAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG was 95% or above. After DEAE, TMAE, Anti-A/Anti-B mix chromatography, ultrafiltration and formulation, the total yield of IgG was 89.6%, the purity was 99.7%, Alb, IgA and IgM were all removed fully, and the osmolarity was 268 mOsmol/kg, meeting the standards. The molecule distribution of IgG monomer and dimer was 99.4%, Anti-A=1:8, Anti-B=1:16, ACA=11%, PKA<10 IU/mL, all meeting the requirements of the Chinese Pharmacopoeia (2010).

### Example 11

A finished product of immunoglobulin IgG concentrate for intravenous injection was prepared by using fraction I+II+III from human plasma as a raw material according to the method in Example 6, and the difference was described as below.

In step (6), after addition of glycine and pH adjusting, the protein solution was incubated at 25 °C under low pH (pH 4.4) for 21 days, filtered through a nano-membrane of 20 nm, followed by mixing and formulating the filtered solution, and filling after sterilization to obtain the immunoglobulin IgG concentrate for intravenous injection.

A total of about 54.15 g total protein was obtained and the product yield was 6.84 g/L plasma. After the Fractogel EMD TMAE anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG was 94% or above. After DEAE, TMAE, Anti-A/Anti-B mix chromatography, ultrafiltration and formulation, the total yield of IgG is 88.7%, the purity was 99.6%, Alb, IgA and IgM were all removed fully, and the osmolarity was 271 mOsmol/kg, meeting the standards. The molecule distribution of IgG monomer and dimer was 99.5%, Anti-A=1:8, Anti-B=1:8, ACA=14%, PKA<10 IU/mL, all meeting the requirements of the Chinese Pharmacopoeia (2010).

### Example 12

A finished product of immunoglobulin IgG concentrate for intravenous injection was prepared by using fraction II+III from human plasma as a raw material according to the method in Example 1, and the difference was described as below.

In step (5), Eshmuno P Anti-A single column was used for affinity chromatography, and the column was filled with Eshmuno P Anti-A affinity gels to a height of 6 cm.

A total of about 56.32 g total protein was obtained and the product yield was 6.98 g/L plasma. After the Fractogel EMD DEAE anion chromatography, the albumin, IgA and IgM in the flow-through solution and washing solution mixture were fully removed. After the Eshmuno P Anti-A affinity chromatography, the product exhibits excellent anti-A removal effect and single-step yield of IgG was 95% or above. After DEAE, TMAE, Anti-A chromatography, ultrafiltration and formulation, the total yield of IgG was 88.4%, the purity was 99.7%, Alb, IgA and IgM were all removed fully, and the osmolarity was 277 mOsmol/kg, meeting the standards. The molecule distribution of IgG monomer and dimer was 99.3%, Anti-A=1:8, Anti-B=1:32, ACA=15%, PKA<10 IU/mL, all meeting the requirements of the Chinese Pharmacopoeia (2010).

### Example 13

A finished product of immunoglobulin IgG concentrate for intravenous injection was prepared by using fraction I+II+III from human plasma as a raw material according to the method in Example 2, and the difference was described as below.

In step (5), Eshmuno P Anti-B single column was used for affinity chromatography, and the column was filled with Eshmuno P Anti-B affinity gels to a height of 25 cm.

A total of about 56.11 g total protein was obtained and the product yield was 6.92 g/L plasma. After the Fractogel EMD DEAE anion chromatography, the albumin, IgA and IgM in the flow-through solution and washing solution mixture were fully removed. After the Eshmuno P Anti-B affinity chromatography, the product exhibits excellent anti-B removal effect and single-step yield of IgG was 95% or above. After DEAE, TMAE, Anti-B chromatography, ultrafiltration and formulation, the total yield of IgG was 88.9%, the purity was 99.6%, Alb, IgA and IgM were all removed fully, and the osmolarity was 272 mOsmol/kg, meeting the standards. The molecule distribution of IgG monomer and dimer is 99.1%, Anti-A=1:32, Anti-B=1:8, ACA=11%, PKA<10 IU/mL, all meeting the requirements of the Chinese Pharmacopoeia (2010).

### Example 14

A finished product of immunoglobulin IgG concentrate for intravenous injection was prepared by using fraction II+III from human plasma as a raw material according to the method in Example 5, and the difference was described as below.

Step (5) was directly performed after completion of step (3), in which Eshmuno P Anti-A single column was used for affinity chromatography and was filled with Eshmuno P Anti-A gels to a height of 6 cm.

A total of about 55.67 g total protein was obtained and the product yield was 6.88 g/L plasma. After the Fractogel EMD DEAE anion chromatography, the albumin, IgA and IgM in the flow-through solution and washing solution mixture were fully removed. After the Eshmuno P Anti-A affinity chromatography, the product exhibits excellent anti-A removal effect and single-step yield of IgG was 95% or above. After DEAE, TMAE, Anti-A chromatography, ultrafiltration and formulation, the total yield of IgG was 89.2%, the purity was 99.6%, Alb, IgA and IgM were all removed fully, and the osmolarity was 278 mOsmol/kg, meeting the standards. The molecule distribution of IgG monomer and dimer was 99.1%, Anti-A=1:4, Anti-B=1:32, ACA=14%, PKA<10 IU/mL, all meeting the requirements of the Chinese Pharmacopoeia (2010).

### Example 15

A finished product of immunoglobulin IgG concentrate for intravenous injection was prepared by using fraction I+II+III from human plasma as a raw material according to the method in Example 6, and the difference was described as below.

Step (4) and step (5) were directly performed after completion of step (2), in which Eshmuno P Anti-B single column was used for affinity chromatography and was filled with Eshmuno P Anti-B gels to a height of 25 cm.

A total of about 56.31 g total protein was obtained and the product yield was 6.98 g/L plasma. After the Fractogel EMD DEAE anion chromatography, the albumin, IgA and IgM in the flow-through solution and washing solution mixture were fully removed. After the Eshmuno P Anti-B affinity chromatography, the product exhibits excellent anti-B removal effect and single-step yield of IgG was 95% or above. After DEAE, TMAE, Anti-B chromatography, ultrafiltration and formulation, the total yield of IgG was 89.2%, the purity was 99.7%, Alb, IgA and IgM were all removed fully, and the osmolarity was 266 mOsmol/kg, meeting the standards. The molecule distribution of IgG monomer and dimer was 99.2%, Anti-A=1:32, Anti-B=1:4, ACA=9%, PKA<10 IU/mL, all meeting the requirements of the Chinese Pharmacopoeia (2010).

### Example 16

An IgG concentrate was prepared by using the raw material and specific method described in Example 1, and the difference was described as below.

In the chromatography A of step (3), Capto Q gels were used to prepare the anion chromatography column, and the chromatography condition was the same as Example 1.

In the chromatography B of step (4), CaptoQ XP gels were used to prepare the anion chromatography column, and the chromatography condition was the same as Example 1.

A total of about 51.16 g total protein was obtained and the product yield was 6.06 g/L plasma. After the Capto Q anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG was 90% or above. After Capto Q, CaptoQ XP and Anti-A/Anti-B mix chromatography, ultrafiltration and formulation, the total yield of IgG was 78.5%, the purity was 96.7%, Alb, IgA and IgM were all removed fully. The molecule distribution of IgG monomer and dimer was 98.1%, Anti-A=1:16, Anti-B=1:16, ACA=14%, PKA<10 IU/mL, all meeting the requirements of the Chinese Pharmacopoeia (2010).

### Example 17

An IgG concentrate was prepared by using the raw material and specific method described in Example 2, and the difference was described as below.

In the chromatography A of step (3), UNO sphere Q gels were used to prepare the anion chromatography column, and the chromatography condition was the same as Example 2.

In the chromatography B of step (4), Nuvia HR Q gels were used to prepare the anion chromatography column, and the chromatography condition was the same as Example 2.

A total of about 50.83 g total protein was obtained and the product yield was 5.98 g/L plasma. After the UNO sphere Q anion chromatography, the albumin, IgA and IgM in the flow-through solution and washing solution mixture were fully removed. After the Eshmuno P Anti-A/Anti-B affinity chromatography, the product exhibits excellent anti-A and anti-B removal effect and single-step yield of IgG was 90% or above. After UNO sphere Q, Nuvia HR Q and Anti-A/Anti-B mix chromatography, ultrafiltration and formulation, the total yield of IgG was 77.6%, the purity was 95.9%, Alb, IgA and IgM were all removed fully. The molecule distribution of IgG monomer and dimer was 98.2%, Anti-A=1:16, Anti-B=1:16, ACA=13%, PKA<10 IU/mL, all meeting the requirements of the Chinese Pharmacopoeia (2010).

### Example 18

A finished product of IgG concentrate was prepared on a pilot scale by using fraction I+II+III according to the production process flow in FIG. 1 and the specific operation steps in Example 1. The yield statistics is shown in Table 2.

**Table 2**

| | Feeding scale: 140 L plasma from healthy human | | | Feeding scale: 200 L plasma from healthy human | | |
|---|---|---|---|---|---|---|
| Yield (kg/ton) | 6.9 | 6.8 | 6.8 | 7.1 | 6.9 | 6.9 |

### Comparative Example 1

A finished product of IgG concentrate for intravenous injection was prepared by using the raw material and method in Example 1, in which the protein content was increased to 30 g/L and the gel load was increased to 800 g precipitates per 400 mL gels. After the Fractogel EMD DEAE anion chromatography, the IgA and IgM in the flow-through solution and washing solution mixture were not removed fully, with 2 to 5% of IgM and IgA remaining in the product.

### Comparative Example 2

A finished product of IgG concentrate for intravenous injection was prepared by using the raw material and method in Example 2, in which the chromatography condition in step (3) was pH 6.50, 25 mM of sodium acetate buffer, 3.0 ms/cm of conductivity and 170 cm/hour of linear rate for equilibration and loading. After the Fractogel EMD DEAE anion chromatography, the IgA and IgM in the flow-through solution and washing solution mixture were not removed fully, with 3 to 6% of IgM and IgA remaining in the product.

### Comparative Example 3

A finished product of IgG concentrate for intravenous injection was prepared by using the raw material and method in Example 5, in which in step (5), a second flow-through solution and washing solution mixture obtained in step (4) was ultrafiltration concentrated with a buffer comprising sodium acetate at pH 4.20 and 60 mM NaCl to a protein content of 80 g/L. The total IgG yield was 68.3%; Alb, IgA and IgM were all removed fully; Anti-A=1:32, Anti-B=1:16; and the osmolarity cannot meet the standard of the Chinese Pharmacopoeia (2010), with natural crystallization visible to the naked eyes.

### Comparative Example 4

A finished product of IgG concentrate for intravenous injection was prepared by using the raw material and method in Example 6, in which in step (5), a second flow-through solution and washing solution mixture obtained in step (4) was diluted with a buffer comprising sodium phosphate at pH 4.10 and 180 mM NaCl to a protein content of 10 g/L. The total IgG yield was 57.1%; Alb, IgA and IgM were all removed fully; Anti-A=1:32, Anti-B=1:16; and the osmolarity cannot meet the standard of the Chinese Pharmacopoeia (2010), with natural crystallization visible to the naked eyes.

### Comparative Example 5

A finished product of IgG concentrate for intravenous injection was prepared by using the raw material and method in Example 1, in which step (6) for the formulation of the finished product was directly performed after completion of step (4), without step (5). The total IgG yield was87.5%, Alb, IgA and IgM were all removed fully and Anti-A=1 :64, Anti-B=1:128, not meeting the standard of the Chinese Pharmacopoeia (2010).

### Comparative Example 6

A finished product of IgG concentrate for intravenous injection was prepared by using the raw material and method in Example 7, in which in step (5), the buffer for chromatography was 10 mM of phosphate buffer saline (PBS) at pH 4.00 and 80 mM NaCl. The total IgG yield was 87.5%; Alb, IgA and IgM were all removed fully; Anti-A=1:4, Anti-B=1:4; and the osmolarity cannot meet the standard of the Chinese Pharmacopoeia (2010), with natural crystallization visible to the naked eyes.

### Comparative Example 7

A finished product of IgG concentrate for intravenous injection was prepared by using the raw material and method in Example 8, in which in step (2), octanoic acid was added dropwise to the dissolved solution to a final concentration of 5 mM within 40 minutes, followed by pressure filtration and subsequent processes, resulting in compaction of Fractogel EMD DEAE gels.

## Claims

1. A method of preparing an intravenous immunoglobulin, comprising steps of:
(1) separating fraction I+II+III or fraction II+III from plasma,
(2) octanoic acid precipitation: precipitating the fraction I+II+III or fraction II+III with octanoic acid, followed by filtering to obtain a clarified liquid,
(3) performing chromatography according to a combination of chromatography A, chromatography B and chromatography C, a combination of chromatography A and chromatography C, or a combination of chromatography B and chromatography C, wherein
chromatography A: filtration concentrating the clarified liquid to obtain a first protein solution in a protein content of 2 to 20 g/L, then passing the first protein solution through an anion chromatography column A under a first chromatography condition of pH 4.50 to 5.50, 5 to 20 mM of sodium acetate buffer, 0.5 to 2.0 ms/cm of conductivity and 60 to 150 cm/hour of linear rate for equilibration and loading,
chromatography B: passing a first flow-through solution and washing solution mixture through an anion chromatography column B under a second chromatography condition of pH 5.50 to 6.50, 5 to 20 mM of sodium acetate buffer, 0.5 to 2.5 ms/cm of conductivity and 60 to 150 cm/hour of linear rate for equilibration and loading,
chromatography C: ultrafiltration concentration of a second flow-through solution the washing solution mixture with a buffer comprising acetate or citrate or phosphate at pH 4.50 to 7.30 and NaCl to obtain a second protein solution in protein content of 20 to 60 g/L, ultrafiltration dialysis of the second protein solution to allow the pH to be consistent with the buffer, thus obtaining a third protein solution, passing the third protein solution through an affinity chromatography column C under an affinity chromatography condition comprising 60 to 150 cm/hour of linear rate under the buffer condition for equilibration and loading,
wherein the anion chromatography column A comprises Fractogel EMD DEAE anion chromatography column, CaptoQ anion chromatography column or UNO sphere Q anion chromatography column,
the anion chromatography column B comprises Fractogel EMD TMAE anion chromatography column, CaptoQ XP anion chromatography column or Nuvia HR Q anion chromatography column, and
the affinity chromatography column C comprises Eshmuno P Anti-A/Anti-B mix affinity chromatography column, Eshmuno P Anti-A affinity chromatography column or Eshmuno P Anti-B affinity chromatography column, and
(4) ultrafiltration concentration or dilution, viral inactivation, formulation and filling to obtain the intravenous immunoglobulin.

2. The method according to claim 1, wherein the anion chromatography column A in the chromatography A is filled with anion gels to a height of 18 to 23cm and has a gel load of 300 to 700 g precipitates per 400 mL gels.

3. The method according to claim 1, wherein the anion chromatography column B in the chromatography B is filled with anion gels to a height of 18 to 23cm and has a gel load of 300 to 700 g precipitates per 400 mL gels.

4. The method according to claim 1, wherein the Eshmuno P Anti-A/Anti-B mix affinity chromatography column, Eshmuno P Anti-A affinity chromatography column or Eshmuno P Anti-B affinity chromatography column in the chromatography C is filled with Eshmuno P Anti-A affinity gels and/or Eshmuno P Anti-B affinity gels to a height of 5 to 25 cm,
the Eshmuno P Anti-A/Anti-B mix affinity chromatography column is filled with Eshmuno P Anti-A affinity gels and Eshmuno P Anti-B affinity gels in a ratio of 1 to 2: 2 to 3.

5. The method according to any one of claims 1 to 4, wherein in the chromatography B, the first flow-through solution and washing solution mixture is subjected to pH adjustment with a buffer, stilled at a temperature of 2 to 8°C for 1 hour or above and filtered to get a supernatant before passing through the anion chromatography column B.

6. The method according to any one of claims 1 to 4, wherein prior to separating fraction I+II+III or fraction II+III from plasma in step (1), the method further comprises treating the healthy human plasma with ethanol at a low temperature to obtain the fraction I+II+III or fraction II+III.

7. The method according to claim 6, wherein after being obtained, the fraction I+II+III or fraction II+III is dissolved with 5 to 25 times of water for injection (WFI) in steps at a temperature of 2 to 25 °C and adjusted to pH 4.00 to 5.00, then stirring for 1 hour or above.

8. The method according to claim 7, wherein after dissolving with WFI in steps, the method further comprises adjusting the dissolved solution with NaOH to pH 4.80 to 5.80, adding the octanoic acid dropwise to a final concentration of 10 to 30 mM, and then adjusting with NaOH back to pH 4.80 to 5.80, followed by stirring for at least 1 hour and filtering to obtain the clarified liquid after octanoic acid precipitation.

9. The method according to claim 1, wherein ultrafiltration concentration or dilution in step (4) further comprises ultrafiltration concentrating or diluting a third flow-through solution and washing solution mixture to obtain a fourth protein solution in protein content of 5 to 100 g/L, adding 1 to 5% (w/w) glycine or proline, and adjusting to pH 3.8 to 4.4 before the formulation.

10. The method according to claim 1, wherein the viral inactivation in step (4) comprises:
filtering through a nano-membrane of 20 nm or 15 nm, combining and sterilizing the filtered solution, and then incubating at 25 °C under low pH, followed by mixing and formulating the resulting solution, and filling after sterilization to obtain the intravenous immunoglobulin, or
incubating at 25 °C under low pH, filtering through a nano-membrane of 20 nm or 15 nm, followed by mixing and formulating the filtered solution, and filling after sterilization to obtain the intravenous immunoglobulin.

11. The method according to claim 1, wherein the octanoic acid in step (2) is of a concentration of 10 to 30 mM.

12. The method according to claim 11, wherein the octanoic acid in step (2) is of a concentration of 22 to 25 mM.

13. The method according to claim 1, wherein
in the chromatography A of step (3),
the first protein solution has a protein content of 5 to 15 g/L,
the anion chromatography column A is Fractogel EMD DEAE anion chromatography column,
the first chromatography condition comprises: pH 4.70 to 5.30, 8 to 16 mM of sodium acetate buffer, 0.8 to 1.8 ms/cm of conductivity and 70 to 120 cm/hour of linear rate for equilibration and loading;
in the chromatography B of step (3),
the anion chromatography column B is Fractogel EMD TMAE anion chromatography column,
the second chromatography condition comprises: pH 5.70 to 6.30, 8 to 16 mM of sodium acetate buffer, 0.8 to 1.8 ms/cm of conductivity and 70 to 100 cm/hour of linear rate for equilibration and loading;
in the chromatography C of step (3),
the second protein solution has a protein content of 30 to 50 g/L,
the buffer comprises 5 to 20 mM of acetate or citrate or phosphate at pH 5.00 to 6.00 and 80 to 150 mM NaCl, and
the affinity chromatography condition comprises 70 to 130 cm/hour of linear rate for equilibration and loading under the buffer condition.

14. The method according to claim 1, wherein the formulation in step (4) is conducted by using glycine or proline.

15. The method according to claim 2, wherein in the chromatography A of step (3), a gel load is 450 to 550 g precipitates per 400 mL gels.

16. The method according to claim 15, wherein the anion chromatography column A is XK50/30 chromatography column.

17. The method according to claim 3, wherein in the chromatography B of step (3), a gel load is 450 to 550 g precipitates per 400 mL gels.

18. The method according to claim 17, wherein the anion chromatography column B is XK50/30 chromatography column.

19. The method according to claim 4, wherein in the affinity chromatography column C, the Eshmuno P Anti-A affinity gels and/or Eshmuno P Anti-B affinity gels filled are of a height of 6 to 20 cm.

20. The method according to claim 19, wherein the affinity chromatography column C is XK 16/20 chromatography column.
